# EUROPEAN PATENT APPLICATION

(11) **EP 2 353 576 A1**
(43) Date of publication of application: **10.08.2011**
(21) Application number: 11001005.5
(22) Date of filing: 08.02.2011
(51) Int. Cl.: A61K 6/00

(54) **Tooth coating material**

(30) Priority: 09.02.2010 JP 2010026344
(71) Applicant: GC Corporation, Itabashi-ku Tokyo 174-8585 (JP)
(72) Inventor: Hinoura, Ko, Nakano-ku Tokyo (JP); Kato, Shinichi, Itabashi-ku Tokyo (JP)
(74) Representative: Müller-Boré & Partner Patentanwälte

(57) **Abstract**

To easily confirm a coating state and covering state of a tooth and coating material on a tooth, the tooth coating material is made to be visually confirmable clearer than surrounding teeth by irradiating a fluorescent agent therein with an ultraviolet or visible ray by a dental light irradiator to excite the fluorescent agent, for example, the tooth coating material containing no water, but including: 0.5 to 20% by weight of a caseinphosphopeptide-amorphouscalciumphosphate complex (CPP-ACP) and/or a casein phosphopeptide-amorphous calcium fluoride phosphate complex (CPP-ACFP), 5 to 70% by weight of a covering film- forming polymer, 5 to 70% by weight of an organic solvent selected so as to contain alcohol, 0.01 to 10% by weight of a fluorine compound, 0.1 to 20% by weight of a thickener, and 0.1 to 3% by weight of the fluorescent agent.

## Description

The present invention relates to a tooth coating material which is applied to a tooth so as to form a film and gives a dental caries preventing effect for a long period of time, wherein a coating state or a covering state of the tooth coating material can be easily confirmed.

As a general tooth strengthening method, a method of coating a fluorine compound to a tooth surface to thereby strengthen enamel and improve acid resistance has been used in general. On the other hand, as a method for previously preventing formation of a dental plaque, a method of blending an antimicrobial agent with a dental composition used for a tooth surface or the inside of an oral cavity has been used (e.g., refer to Japanese Patent Application Laid-Open No. H4-189661, Japanese Patent Application Laid-Open No.H4-346905, Japanese Patent Application Laid-Open No. H5-126398, Japanese Patent Application Laid-Open No. H5-015435, Japanese Patent Application Laid-Open No. H6-192060, Japanese Patent Application Laid-Open No. H7-206621, Japanese Patent Application Laid-Open No. H7-215814, Japanese Translation of PCT Publication No. 2006-507361, Japanese Translation of PCT Publication No. 2006-505303, Japanese Patent Application Laid-Open No. 2007-254300, and Japanese Patent Application Laid-Open No. 2007-269637).

There is also a method of coating, to a tooth surface, a composition blended with a fluorine compound such as a casein phosphopeptide-amorphous calcium phosphate complex (hereinafter referred to as CPP-ACP) as a fluorine compound, and thereby forming a covering film including the fluorine compound and strengthening a dentin. The CPP-ACP includes a complex of peptide originating in protein (casein) of a cow's milk and an inorganic material (amorphous calcium phosphate). Because of including calcium and phosphorus, which are inorganic components of a tooth, in its constituent, the CPP-ACP can more effectively promote recalcification of a tooth as a time of holding the CPP-ACP on the tooth surface is longer. Further, a casein phosphopeptide-amorphous calcium fluoride phosphate complex (it will be called CPP-ACFP below), which includes fluoride, has also effect for preventing dental caries (e.g., refer to Japanese Translation of PCT Publication No. 2002-500626, Japanese Translation of PCT Publication No. 2002-540129, and Japanese Patent Application Laid-Open No. 2005-145952.).

However, since such tooth coating materials as above mentioned are worn over time, the effect for preventing dental caries is lost in a while after coating the materials. The tooth coating materials are transparent so as not to affect aesthetic property in an oral cavity, or a color tone of the composition is ivory or white similar to a color of a tooth. Thus, when the tooth coating material is worn, there is a problem that it is very difficult to visually confirm the covering state. If the wearing state of the tooth coating material cannot be properly determined, there will be a time-lag up to next covering, so that dental caries cannot be prevented sufficiently. Further, even if unevenness of covering occurs at a time of coating, an operator can not notice it, so that an effect of the tooth coating material may become insufficient.

Accordingly, an objective of the present invention is to provide a tooth coating material, of which a covering state and a coating state on a tooth can be easily confirmed.

Present inventors carried out earnest works to solve the aforementioned problems, and as a result, they found out the followings to complete the present invention. When a fluorescent agent is blended with a conventional tooth coating material so that the tooth coating material has a higher fluorescence effect than a natural tooth, the tooth coating material can be visually confirmed clearer than surrounding teeth by irradiating the fluorescent agent with ultraviolet rays or a visible ray by a dental light irradiator and exciting the fluorescent agent. Therefore, the covering state can be confirmed easily and clearly.

That is, according to an aspect of the present invention, a tooth coating material including a fluorine compound and being used for coating a tooth to prevent dental caries includes 0.1 to 3% by weight of a fluorescent agent.

The covering state of tooth coating material according to the present invention can be confirmed easily and clearly by irradiating the fluorescent agent with ultraviolet rays or a visible ray by a dental light irradiator and exciting the fluorescent agent.

Since a tooth coating material of the present invention is produced by blending a fluorescent agent with a conventional tooth coating material, a conventional blend of the tooth coating material for preventing dental caries can be used without any restrictions. Specifically, the present invention is effective for a tooth coating material having a comparatively low covering ability, without containing polymerizable compounds for polymerization hardening as its constituents, for example, methacrylate, acrylate and the like. More specifically, the present invention is effective for a tooth coating material which has a comparatively low covering ability because of not being polymerized and hardened, and needs to keep in a coating state on a tooth surface, where such a tooth coating material is, for example, a composition not containing water but containing a caseinphosphopeptide-amorphouscalciumphosphate complex (CPP-ACP) and/or a casein phosphopeptide-amorphous calcium fluoride phosphate complex (CPP-ACFP).

As the fluorescent agent used for the present invention, a fluorescent agent making a fluorescent reaction by a dental light irradiator can be used. More specifically, an inorganic fluorescent pigment, e.g., sulfides of alkali earth metals such as calcium tungstate, calcium and magnesium arsenates, barium silicate, calcium phosphate, zinc calcium phosphate and the like, silicate, phosphate, tungstate, an organic fluorescent pigment, an organic fluorescent dye, e.g., a phthalic acid derivative (diethyl-2,5-dihydroxyterephthalate, o-phthalaldehyde), a thiophene derivative (2,5-bis(5'-t-butylbenzoxazolyl-2')thiophene, 2,5-bis(6,6'-bis(tert-butyl)-benzoxazole-2-yl)thiophene), a coumarin derivative (3-phenyl-7-(4-methyl-5-phenyl-1,2,3-triazole-2-yl)coumarin, 3-phenyl-7-(2H-naphtho[1,2-d]-triazole-2-yl) coumarin), a naphthalimide derivative (N-methyl-5-methoxynaphthalimide), a stilbene derivative (4,4'-bis (diphenyltriazinyl) stilbene, 4,4'-bis(benzoxazole-2-yl)stilbene), a benzothiazole derivative, and the like can be used. In these materials, the phthalic acid derivative and the thiophene derivative are preferable.

It is preferable that the fluorescent agent itself is colorless and transparent as much as possible. Considering this point, a wavelength peak of the fluorescent agent is preferably 420nm or less, and more preferably 320 to 400nm. As for a content of the fluorescent agent, 0.1 to 3% by weight of the fluorescent agent needs to be blended in the whole tooth coating material, and 0.5 to 3% by weight is preferable. The fluorescent agent has been conventionally blended, in some time, in dental compositions such as a composite resin, cement and the like, for adjusting a color tone to be similar to a tooth color tone. However, in such a case, a blending ratio was less than 0.1% by weight for adjusting the color tone to be similar to the tooth color tone.

A casein phosphopeptide-amorphous calcium phosphate complex (CPP-ACP) and/or a casein phosphopeptide-amorphous calcium fluoride phosphate complex (CPP-ACFP) used for the present invention are not limited especially when these are permitted as a food or in a dental field. For example, Japanese Translation of PCT Publication No. 2002-500626 discloses a method for preparing the CPP-ACP and/or CPP-ACFP. In this method, phosphopeptide is mixed with calcium, inorganic phosphoric acid, and arbitrarily fluorine, and the mixture is filtrated and dried. The composition prepared by this method is preferably used from view points of dental caries preventing effect and degree of crystallinity.

The blending amount of the CPP-ACP and/or the CPP-ACFP is 0.5 to 20% by weight with respect to the whole tooth coating material, and is preferably 1 to 12% by weight. If the amount is less than 0.5% by weight, calcium and phosphorus is supplied insufficient, and thus a dental caries preventing effect cannot be obtained. If the amount exceeds 20% by weight, viscosity of the tooth coating material becomes too high, and storage ability decreases.

A covering film- forming polymer blended in the tooth coating material is a necessary component for forming a film on the tooth after coating of the tooth coating material to a tooth, keeping the film stably on the tooth, and continuously exercising various dental caries preventing effects. As for the covering film- forming polymer, natural materials such as shellac, rosin, pullulan, zein, carnauba wax, beeswax, hemilose, and gelatin, and organic synthetic materials such as polyethylene glycol, polypropylene glycol, polyethylene oxide, polyvinyl alcohol, polyvinyl acetate, polyvinyl chloride, a vinyl chloride-vinyl acetate copolymer, polyacrylic acid, polymaleic acid, an alkyl acrylate polymer, an alkyl methacrylate polymer, a methoxymethylene /maleic anhydride copolymer, a polyethylene glycol / methylenediisocyanatocyclohexane copolymer and a polypropylene glycol / methylenediisocyanatocyclohexane copolymer, for example, can be used. Of course, two or more kinds can be combined to be used.

The blending amount of the covering film-forming polymer is 5 to 70% by weight with respect to the whole tooth coating material and is preferably 10 to 60% by weight. If the amount is less than 5% by weight, the covering film formed after coating is insufficient, and thus there is a problem that the covering film abrades easily. If the amount exceeds 70% by weight, storage stability of the tooth coating material decreases.

After coating the tooth coating material to the tooth, the tooth coating material is naturally dried or dried by air blow and a covering film is formed on the tooth surface. In order to properly carrying out coating, drying, and film formation, it is necessary for the tooth coating material to include an organic solvent selected so as to contain alcohol as a volatile component.

Alcohol as the organic solvent is indispensable. As for the alcohol, ethanol, methanol, propanol, isopropanol, butanol, isobutanol, pentanol, isopentanol, oleyl alcohol, octanol, benzyl alcohol, methyl cellosolve, and ethyl cellosolve, for example, can be used. Further, two or more kinds of alcohols can be combined to be used.

The blending amount of the alcohol is 30 to 95% by weight with respect to the whole organic solvent, and is preferably 45 to 70% by weight. If the amount is less than 30% by weight, sufficient volatile effects for forming the covering film cannot be obtained. If the amount exceeds 95% by weight, viscosity of the tooth coating material before coating tends to be too low.

The organic solvent used in the present invention other than the alcohol can be one kind or a combination of two or more kinds selected from acetone, methyl ethyl ketone, methyl isobutyl ketone, styrene, n-pentane, isopentane, tetramethyl methane, n-hexane, cyclohexane, n-heptane, 2-methylhexane, 3-methylhexane, dimethylpentane, trimethylbutane, butyl glycol, methyl diglycol, ethyl diglycol, butyl diglycol, dimethyl diglycol, 1-methoxy-2-propanol, methyl dipropylene glycol, 3-methoxybutanol, tetrahydrofuran, ethyl acetate, propyl acetate, isopropylacetate, butyl acetate, isobutyl acetate, butyl cellosolve acetate, ethyl carbitol acetate, and the like.

The blending amount of the organic solvent is 5 to 70% by weight with respect to the whole tooth coating material, and is preferably 10 to 50% by weight. If the amount is less than 5% by weight, the covering film-forming polymer is hardly dissolved with the organic solvent, so that a coating operation becomes hard. If the amount exceeds 70% by weight, preservation stability of the tooth coating material decreases.

The tooth coating material includes a fluorine compound for giving a more recalcification effect. The blending amount of the fluorine compound is preferably 0.01 to 10% by weight, and more preferably 0.1 to 6% by weight. If the amount is less than 0.01% by weight, the sufficient recalcification effect is hardly obtained. If the amount exceeds 10% by weight, there is a problem in safety to a human body. As for the fluorine compound, one kind or a combination of two or more kinds selected from sodium fluoride, tin fluoride, and monofluoro sodium phosphate can be used.

It is necessary for the tooth coating material according to the present invention to add a proper thickener for improving operativity of the tooth coating material and stabilizing blended materials in the tooth coating material so as to suppress liquid separation. The thickener can be one kind or a combination of two or more kinds selected from aerogel, methyl cellulose, carboxymethylcellulose, carboxymethylcellulose sodium, carboxymethylcellulose calcium, carboxymethylcellulose pottasium, hydroxyethyl cellulose, hydroxypropyl cellulose, polyvinyl pyrrolidone, acacia gum, Arabian gum, guar gum, carob bean gum, tara gum, tamarind seed gum, tragacanth gum, karaya gum, carrageenan, chitin, chitosan, chitosamine, xanthan gum, gellan gum, curdlan, carbopol, Lucentite, sodium alginate, propylene glycol alginate, sodium starch glycolate, sodium starch phosphate, sodium polyacrylate, and the like.

The blending amount of the thickener with respect to the whole tooth coating material is 0.1 to 20% by weight, and is preferably 0.5 to 10% by weight. If the amount is less than 0.1% by weight, preservation stability of the tooth coating material decreases. If the amount exceeds 20% by weight, viscosity of the tooth coating material becomes too high, so that the tooth coating material is hardly coated on a tooth surface.

The tooth coating material according to the present invention can include an antimicrobial agent in order to give a sterilization effect to a tooth face by coating of the tooth coating material to the tooth. The antimicrobial agent can be one kind or a combination of two or more kinds selected from sodium azulenesulfonate, ε-aminocaproic acid, allantoin, allantoin chlorohydroxyaluminum, allantoin dihydroxyaluminum, epidihydrocholesterol, dihydroxycholesterol, glycyrrhizinic acid, diammonium glycyrrhizinate, disodium glycyrrhizinate, trisodium glycyrrhizinate, dipotassium glycyrrhizinate, monoammonium glycyrrhizinate, chlorhexidine gluconate, β-glycyrrhetinic acid, isopropyl methyl phenol, cetylpyridinium chloride, dequalinium chloride, benzalkonium chloride, a benzalkonium chloride liquid, benzethonium chloride, a benzethonium chloride liquid, an alkyldiaminoethylglycine hydrochloride liquid, chlorhexidine hydrochloride, chlorhexidine gluconate, triclosan, pyridoxine hydrochloride, acetic acid d1-α-tocopherol, nicotinic acid d1-α-tocopherol, zeolite, methyl paraoxy-benzoate, ethyl paraoxy-benzoate, propyl paraoxy-benzoate, butyl paraoxy-benzoate, benzyl paraoxy-benzoate, disodium dihydrogen pyrophosphate, sodium pyrophosphate, anhydrous sodium pyrophosphate, sodium hydrogen phosphate, trisodium phosphate, sodium polyphosphate, stearyl dimethyl benzyl ammonium chloride, lauryl dimethyl benzyl ammonium chloride, distearyl dimethyl ammonium chloride, stearyl trimethyl ammonium chloride, cetyl trimethyl ammonium chloride, lauryl trimethyl ammonium chloride, stearyl trimethyl ammonium bromide, cetyl trimethyl ammonium bromide, benzalkonium chloride, benzethonium chloride, dequalinium chloride, chlorhexidine chloride, chlorhexidine gluconate, chlorhexidine acetate, hexetidine, alkyldiaminoethylglycine hydrochloride, domiphen bromide, lauroylcolaminoformylmethylpyridinium chloride, cetyltrimethylammonium saccharin, triclosan, 2,2'-dihydroxy-5,5'-dibromo-diphenylether, 4',5-dibromosalicylanilide, 3,4',5-trichlorosalicylamide, 3,4',4-trichlorocarbanilide, 3-trifluoromethyl-4,4-dichlorocarbanilide, 2-isopropyl-5-methyl-phenol(thymol), 2-methoxy-4-(2-propenyl)-phenol(eugenol), methyl-p-chlorophenol, ethyl-p-chlorophenol, hexylresorcinol, methyl-resorcinol, methyl-resorcinol, dodecyldiaminoethylglycine, benzoate, lauroyl sarcosine, lauryl sulfate, and the like.

As for the antimicrobial agent, the blending amount is preferably within a range from 0.001 to 3% by weight, and more preferably 0.01 to 0.5% by weight. If the amount is less than 0.001% by weight, the sufficient antimicrobial effect is hardly obtained. If the amount exceeds 3% by weight, there is a problem in safety to a human body.

As for the state of the tooth coating material according to the present invention, any one of a liquid state, a gel state, a paste state, and a cream state can be used. As for a method for coating the tooth coating material to a tooth, a coating method using a brush or a swab, or a spraying method using a spray can be considered and any methods can be used.

In addition, the tooth coating material according to the present invention can properly include additives such as a coloring agent, a bleaching agent and the like.

### [Example]

The present invention will be described in detail below with examples and comparative examples, but the present invention is not limited to these examples.

Blending ratios of compositions of Examples 1 to 5 and Comparative examples 1 and 2 are shown in Table 1 collectively. Units of values in Table 1 are weight parts.

### <Confirmation and evaluation of persistence on tooth face - Abrasion test>

(1) A cow's tooth was embedded with a dental resin and a cow's tooth enamel was mirror polished by a water-resistant polishing paper.
(2) A tooth coating material blended with compositions shown in Table 1 was applied to the mirror polished cow' s tooth enamel with a brush as to cover the whole tooth face, and dried with low pressure air.
(3) The tooth coating material was held for 1 hour at a temperature of 37°C and a humidity of 100%, and thereafter subjected to an abrasion test of 8000 times by a brush abrasion testing machine. Then, the persistence degree of the covering film on the tooth face was visually confirmed. After that, the covering film is light-irradiated by a dental light irradiator (the product name: LABOLIGHT LV-II, produced by GC Corporation) and the persistence degree was confirmed again.

Good: The covering state could be easily confirmed Bad: The covering state was hardly confirmed.

**<Table 1>**

| | | **Example 1** | **Example 2** | **Example 3** | **Example 4** | **Example 5** | **Comparative example 1** | **Comparative example 2** |
|---|---|---|---|---|---|---|---|---|
| CPP-ACP and/or CPP-ACFP | | | | | | | | |
| | CPP-ACP | | | 2 | 5 | 3 | | |
| | CPP-ACFP | | 5 | 2 | | 4 | 2 | 2 |
| Coating film-forming polymer | | | | | | | | |
| | Shellac | 30 | | | | | | |
| | Zein | | 25 | 12 | | | | |
| | Rosin | | | 20 | | | | |
| | Polyvinyl acetate | | | | 45 | | 47 | |
| | Alkyl methacrylate polymer | | | | | 35 | | 32 |
| Organic solvent selected so as to contain alcohol | | | | | | | | |
| Alcohol | | | | | | | | |
| | Ethanol | 55 | 40 | | 28 | | | 20 |
| | Isopropanol | | | 40 | | | | |
| | Oleyl alcohol | | | | | 44 | | |
| Organic solvent other than alcohol | | | | | | | | |
| | Isopentane | 65 | | | | | | |
| | n-hexane | | 20 | | | 10 | 46 | |
| | Ethyl diglycol | | | 17 5 | | | | |
| | Tetrahydrofuran | | | | 15 | | | |
| Fluonne compound | | | | | | | | |
| | Sodium fluoride | 1 | | | 3 | | | |
| | Monofluoro sodium phosphate | | 42 | | | 2 | | 3 |
| | Tin fluoride | | | 2 | | | 2 | |
| Thickener | | | | | | | | |
| | Aerosil R972 | | | | | | | 3 |
| | Aerosil A200 | 4 | | | | | | |
| | Hydroxypropyl cellulose | | | 2 | | 1 | | |
| | Polyvinyl pyrrolidone | | 4 | | | | 2 | |
| | Sodium alginate | | | | 2 | | | |
| Antimicrobial agent | | | | | | | | |
| | Chlorhexidine hydrochlonde | | | | | | | |
| | Cetylpyridinium chlonde | | | | | | | |
| Flurescent agent | | | | | | | | |
| | 2,5-bis(5'-t-butylbenzoxazolyl-2')thiophene | | 18 | 15 | | | 0 05 | |
| | 3-phenyl-7-(4-methyl-5-phenyl-1,2,3-triazola-2-yl)coumann | 2 5 | | | 2 | 1 | | |
| Other additives | | | | | | | | |
| | Water | | | | | | | 40 |
| | Xylitol | 1 | | 1 | | | 0 95 | |
| Total | | 100 0 | 100 0 | 100 0 | 100 0 | 100 0 | 100 0 | 100 0 |
| Abrasion test [persistence after testing 8000 times] | | Good | Good | Good | Good | Good | Bad | Bad |

## Claims

1. A tooth coating material containing a fluorine compound and being used by applying to a tooth for preventing dental caries, wherein 0.1 to 3% by weight of a fluorescent agent is contained.

2. A tooth coating material used by applying to a tooth, which does not contain water and comprises 0.5 to 20% by weight of a casein phosphopeptide-amorphous calcium phosphate complex (CPP-ACP) and/or a casein phosphopeptide-amorphous calcium fluoride phosphate complex (CPP-ACFP); 5 to 70% by weight of a covering film- forming polymer; 5 to 70% by weight of an organic solvent selected so as to contain alcohol; 0.01 to 10% by weight of a fluorine compound; and 0.1 to 20% by weight of a thickener, wherein the tooth coating material contains 0.1 to 3% by weight of a fluorescent agent.

3. The tooth coating material as claimed in claim 1 or 2, wherein the covering film-forming polymer is one kind or a combination of two or more kinds selected from shellac, rosin, pullulan, zein,
carnauba wax, beeswax, hemilose, gelatin, polyethylene glycol, polypropylene glycol, polyethylene oxide, polyvinyl alcohol, polyvinyl acetate, polyvinyl chloride, a vinyl chloride-vinyl acetate copolymer, polyacrylic acid, polymaleic acid, an alkyl acrylate polymer, an alkyl methacrylate polymer, a methoxymethylene / maleic anhydride copolymer, a polyethylene glycol /methylenediisocyanatocyclohexane copolymer, and a polypropylene glycol / methylenediisocyanatocyclohexane copolymer.

4. The tooth coating material as claimed in any of claims 1 to 3, wherein the fluorescent agent is one kind or a combination of two or more kinds selected from diethyl-2, 5-dihydroxyterephthalate, o-phthalaldehyde, 2,5-bis(5'-t-butylbenzoxazolyl-2')thiophene, 2,5-bis (6,6'-bis (tert-butyl) -benzoxazole-2-yl) thiophene, 3-phenyl-7-(4-methyl-5-phenyl-1,2,3-triazole-2-yl)coumarin, 3-phenyl-7-(2H-naphtho[1,2-d]-triazole-2-yl)coumarin, N-methyl-5-methoxynaphthalimide, 4,4'-bis(diphenyltriazinyl)stilbene, 4,4'-bis(benzoxazole-2-yl)stilbene, benzothiazole.
